# EUROPEAN PATENT APPLICATION

(11) **EP 1 116 515 A2**
(43) Date of publication of application: **18.07.2001**
(21) Application number: 00128675.6
(22) Date of filing: 29.12.2000
(51) Int. Cl.: B01J 13/02, A61K 9/50, A61K 7/46, C11D 3/50, A23L 1/22

(54) **Encapsulated liquid**

(30) Priority: 11.01.2000 EP 00100464
(71) Applicant: Givaudan SA, 1214 Vernier-Genève (CH)
(72) Inventor: Ubbink, Johan, Bernhard, 8047 Zürich (CH); Quellet, Christian, 2502 Biel (CH); Taschi, Marc, 5607 Hägglingen (CH)
(74) Representative: Patentanwälte Schaad, Balass, Menzl & Partner AG

(57) **Abstract**

Disclosed is a method for the preparation of a composite material consisting of very fine inclusions of liquid, moderately to strongly hydrophobic active ingredients homogeneously dispersed in a thermoplastic matrix. The interface of the inclusions is fully covered by an emulsifier film.

## Description

This invention refers to the field of encapsulation of active ingredients into a solid matrix of biological or synthetic origin.

Encapsulation is an important method to protect volatile, chemically reactive or otherwise sensitive active ingredients from evaporation and chemical degradation and to reduce the amount of the active ingredient necessary in an application. Well-known are pharmaceutical controlled release applications, in which the dosage of a drug is optimized and possible adverse effects are minimized. Also, suitable encapsulation and controlled release devices often reduce the cost, as the amount of the (expensive) ingredient to be administered may often be substantially reduced. Other fields in which encapsulation and controlled release find successful application are in agriculture, where encapsulated fertilizers and pesticides reduce both environmental impact and cost of application, and in the flavor and fragrance industries, where delivery systems minimize loss of flavor and fragrance compounds during storage and processing and increase the performance of the flavor or the perfume in the end product or application.

For optimal chemical and mechanical stability and optimal controlled release properties, it is desired that the encapsulants (the ingredients to be protected) are dispersed in the matrix in the form of very fine and uniformly dispersed inclusions. This is particularly important in case of liquid encapsulants, where small inclusion size is preferred in order to limit the amount of so-called surface oil upon fracture of the matrix. High amounts of surface oil are undesirable because of release, rapid volatilization or chemical reaction of the free oil and because it causes a granulated product to stick together. One important limitation of the methods used until now in the field of extrusion encapsulation is that a small inclusion size of liquid, hydrophobic ingredients in a solid, hydrophilic matrix is difficult to obtain in a controlled way, especially when the viscosities of both encapsulant and matrix differ strongly. Usually, the materials obtained are coarse and heterogeneous. Coarse and heterogeneous materials are highly non-ideal as far as the encapsulation of volatiles, reactive and otherwise sensitive compounds is concerned. Such materials loose a high amount of encapsulant upon grinding or breaking, which results in a high level of surface oil. This considerably lowers the retention of the encapsulants and is detrimental to the chemical stability of the active ingredients. Moreover, the presence of large inclusions lowers the mechanical strength of the extrudate. Finally, the release of encapsulants from heterogeneous systems may be difficult to control. A further problem with the encapsulation by extrusion of volatile, hydrophobic, liquid active ingredients is that the loss of active ingredients during processing is often substantial because of excessive volatilization of the active ingredients.

The US-A-4232047 discloses a method for the preparation of a food supplement concentrate in a dense glassy extrudate. The encapsulation matrix forming the extruded glass consists of starch, protein, flour, modified starch, gum and mixtures thereof. The edible active agent is mixed with the matrix and a limited quantity of water and extruded. After extruding and drying, the extrudate can be ground to the appropriate particle sizes. The process suffers from several serious disadvantages, however. First, matrices containing starches need high temperatures and pressures and substantial amounts of water to be processed. The active ingredient may rapidly degrade or evaporate under these conditions. Second, in case of liquid, hydrophobic compounds a coarse and heterogeneous product giving high amounts of surface oil upon grinding is obtained.

The WO-A-85/04074, discloses a method for the encapsulation of an insect-controlling material in a starch matrix by extrusion. To avoid high temperatures in the extruder barrel the starch is pregelatinized prior to the encapsulation step in the extruder. The insect-controlling material is mixed with the matrix composition prior to the extrusion step. Thus the method described is an expensive two-step process and when applied to encapsulate liquid, hydrophobic active ingredients, the material obtained would be coarse and heterogeneous unless the active ingredient dissolves in the matrix composition.

The US-A-5183690, discloses encapsulating biologically active agents in a starch matrix by means of a continuous extrusion process. In a first step the biologically active ingredients are uniformly dispersed in an aqueous solution of the dispersed starch. It is mentioned that the order of combining the various components of the formulation is not critical and that it may be conducted in whatever manner best facilitates the process. To obtain acceptable results a very high preparatory skill is needed but normally the process results in coarse and heterogeneous materials with poor characteristics.

In DE 4002257, a method for the encapsulation of active components in starch is described. First, a premix containing starch, water, plasticizers like glycerol and triglycerides, and emulsifiers is extruded in order to gelatinize the starch. The active ingredient is mixed with the pregelatining starch matrix and re-extruded. This two step extrusion process thus allows different processing routes for the matrix and the active ingredients. One of the drawbacks is that it is a two-step process, which is expensive. More significantly, coarse and heterogeneous materials are obtained which do not have the desired properties.

In F.Z. Saleeb; J.L. Cavallo; S. Vidal, *Dev. Food Sci.* **29**, 651 (1992), methods are described for the encapsulation of flavors in a carbohydrate matrix by extrusion. The flavors to be encapsulated are either mixed with the matrix prior to processing, or introduced into the extruder barrel by a feed port. The flavor losses are very high, even when the flavor is injected into the extruder barrel. Furthermore, the structure of the materials is coarse and heterogeneous, as witnessed by the electron micrographs published in the paper.

The US-A 5 087 461 discloses a process for producing compositions containing volatile and/or labile components. These components are first encapsulated by spray-drying. The spray-dried powder is subsequently encapsulated in an extruded glassy matrix. During spray-drying a substantial loss of volatiles is observed and the two step process is expensive. The maximum attainable load of active ingredients in the composition by this process is low. Moreover, the material obtained is very coarse and heterogeneous with high local concentrations of flavor because the spray-dried particles are rather large.

In W.M. Doane, *Ind. Crops and Products* **1***,* 83-87 (1993), various methods are described by which pesticides can be encapsulated in a starch matrix. No other ingredients than starch and water are necessary for the matrix. The pesticides are either added to the premix, or introduced into the extruder barrel after a gelatinization step. Again, for active ingredients which do not readily mix on a molecular scale with the matrix materials, the structure of the obtained composition will be coarse and heterogeneous. For volatile compounds, the efficiency of encapsulation will be low because of excessive volatilization during the extrusion step.

A.N.R. Kollengode, M.A. Hanna and S. Cuppett, J. *Food Sci*. **61**, 985 (1996), discuss volatile retention in extruded corn starch as influenced by the method of addition. The volatiles are either mixed with the matrix premix or directly injected in the extruder barrel. The volatile retention is enhanced when they are injected into the extuder barrel as compared to mixing with the feed. In both cases, volatile retention is low, although the method of direct injection of the volatiles into the extruder barrel generally gives somewhat better results.

In the WO-A-98/18610 preparation of solid controlled release particles is described by extruding a matrix premix containing a plastizible matrix material, as e.g. starches, a hydrophilic or hydrophobic release-rate controlling agent and at least one plasticizer, as for instance water. Optionally, both the extruded material and the active ingredient may be coated with a film-forming agent to control the release rate of the active ingredients, a main variable being the thickness of the coating. All examples refer to solid active ingredients. The release-rate controlling agent is added to tune the release properties in such a way that significant release of active ingredients occurs only in the stomach or in the intestines.

In A.N.R. Kollengode; M.A. Hanna, *Cereal. Chem.* **74,** 396 (1997), a two step process is described. In the first step a matrix premix based on corn starch is extruded in order pregelatinize the starch. In the second step, the pregelatinized matrix is re-extruded with volatile flavor compounds as encapsulants. A significant loss of volatile active ingredients during encapsulation can not be avoided. Another drawback is that the process consists of two steps, which makes it expensive and cumbersome.

It is an object of the invention to overcome the shortcomings of the above prior art.

One of the objects of the invention is to reduce the losses of active ingredients during processing.

It is a further object of the invention to provide for a material in which liquid, active ingredients are encapsulated in the form of very fine inclusions uniformly dispersed through a hydrophilic matrix.

Another object of the invention is to improve the efficiency of encapsulation of volatile and/or sensitive active ingredients.

A further object of the invention is to improve the stability of the inclusions against deformation and fracture of the extrudate. In particular, it is intended to reduce the amount of free surface oil upon fracture of the extrudate.

Another object of the invention is to provide a method, which allows the use of a broader selection of matrix materials and encapsulants of varying viscosities.

A further object of the invention is to provide a controlled release system for a liquid, hydrophobic active ingredient, in particular fragrances, of which the release is triggered by an increase in water activity and optionally temperature.

A further object of the invention is to provide a controlled release system for flavors which releases the flavor compounds in the mouth during consumption of a food product and/or into a food product during processing and/or storage.

It has been surprisingly found that by introducing the liquid active ingredient in the form of water-in-oil emulsion directly into the hydrophilic matrix, e.g. in an extruder barrel, a uniform distribution of the liquid in the matrix is obtained.

Therefore the present invention refers to a composite material comprising a thermoplastic hydrophilic matrix in which a liquid active ingredient in form of a water-in-oil emulsion is encapsulated in the form of very fine and uniformly distributed droplets.

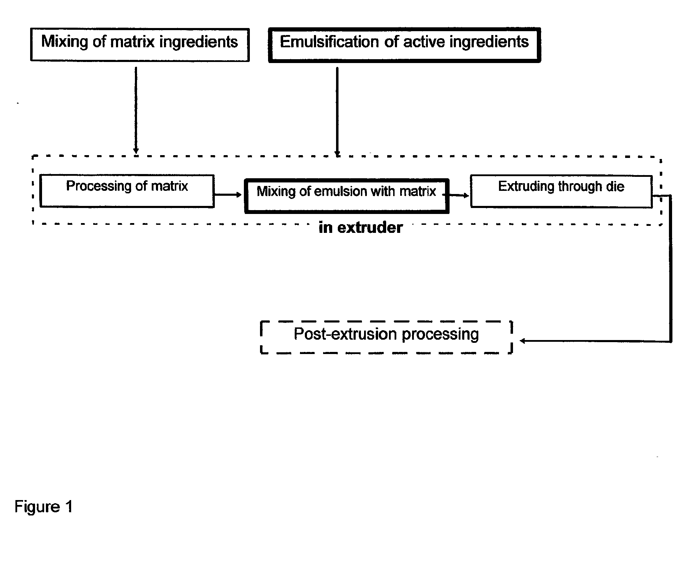

The composite material of the invention can be prepared by emulsifying the liquid, hydrophobic active ingredients to form an oil-in-water (O/W) emulsion. By emulsifying the active ingredient to form an O/W emulsion, the oil droplets are fully covered by the emulsifier film and the droplet size which is obtained is very small. Because of the small size and the neat emulsifier film, the droplets are stable and their structure is not significantly influenced during the extrusion process. The emulsion obtained can be introduced into the barrel of an extruder containing the pre-processed matrix composition, where the emulsion droplets are mixed with the matrix composition and the mixture is extruded through a die (see also figure 1). Alternatively the emulsified active ingredients are added to the matrix premix and the premix is subsequently extruded.

The material of which the matrix surrounding the droplets is made generally contains at least one slightly to strongly hydrophilic polymer or oligomer, of biological or synthetic origin. The polymeric fraction in the matrix premix may vary between 50% and 100% w/w. Optionally, solvents or plasticizers are added, amounting to 1% and 50% w/w of matrix premix. Up to 30% w/w of further additives can be present in the matrix premix. The encapsulated active ingredients are dispersed through the matrix in the form of small inclusions, being in form of droplets, which are stabilized by the emulsifier film. The size of these inclusions is largely determined by the composition of the emulsion and the size distribution of the emulsion droplets and is only weakly influenced by the extrusion process, and typically comprises the range between 0.01 µm and 2 µm, preferably between 0.05 µm and 1 µm. The load of active ingredients in the final product may vary between 1 and 50% w/w, preferably between 5 and 30% w/w.

The active ingredients are emulsified prior to introduction into the matrix composition. The emulsion contains the active ingredients, water and at least one emulsifier. Instead of water, any other hydrophilic solvent can be used. The emulsion contains 5 to 80% w/w, preferably 30 to 60% w/w active ingredients, 10 to 90% w/w, preferably 15 to 40% w/w water, 0.5 to 10% w/w emulsifier and 0 to 10% w/w additives. The O/W emulsion containing the active ingredients may be prepared by any of the well-known techniques. For instance, an O/W emulsion may be prepared by blending the active ingredients with water and a suitable emulsifier by means of a high speed homogenizator. For lab-scale trials, the Ultra-Turrax T25 (IKA Labortechnik) has proved to be very useful. In case of volatile or temperature-sensitive active ingredients, the emulsion mixture can be cooled during the emulsification process, e.g. by putting the container in an ice-bath. Compounds which easily oxidize can be protected by using an inert atmosphere above the emulsion mixture, for instance nitrogen. The viscosity of the emulsion is such that the emulsion can still be mixed with the matrix premix or fed in to the extruder barrel via a feed port. The viscosity typically ranges from 10 cP to 10⁶ cP, preferably from 50 cP to 10⁵ cP as measured by standard rheological techniques. Often, the lower the amount of water, the better the structure of the product obtained and the retention of active ingredients in the final product. This helps to minimize the loss of active ingredients during later processing steps, but is still such that the viscosity of the emulsion is not too high for further processing.

The composite material is prepared by processing the matrix premix in a conventional single or double screw extruder. The emulsion containing the active ingredients may either be added to the premix or introduced into the extruder barrel by a feed port. The first method may be used if the premix is a granular, thermoplastic material with a relatively low melting point or glass-transition point. Usually, however, the latter method is preferable as the matrix premix often needs more extensive processing, usually under relatively harsh conditions which cause the active ingredients to evaporate or to chemically degrade.

The flexibility of extrusion as a technique for encapsulating liquid active ingredients is optimally exploited if the main part of the extruder barrel, closest to the raw materials feed, is used for processing the matrix premix under conditions which induce the optimal matrix structure for the application. In the latter part of the extruder barrel, close to the die, the emulsion containing the active ingredients is introduced by a feed port and mixed with the processed, but still moldable matrix composition. The mixture is extruded through a die and optionally subjected to a post-extrusion step to reduce the particle size.

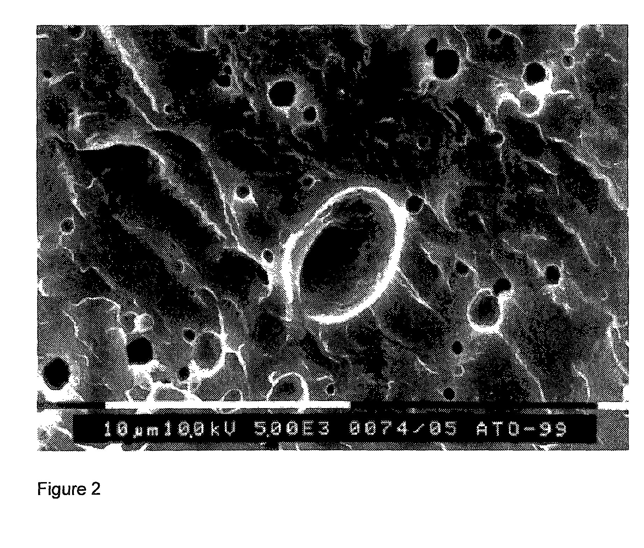

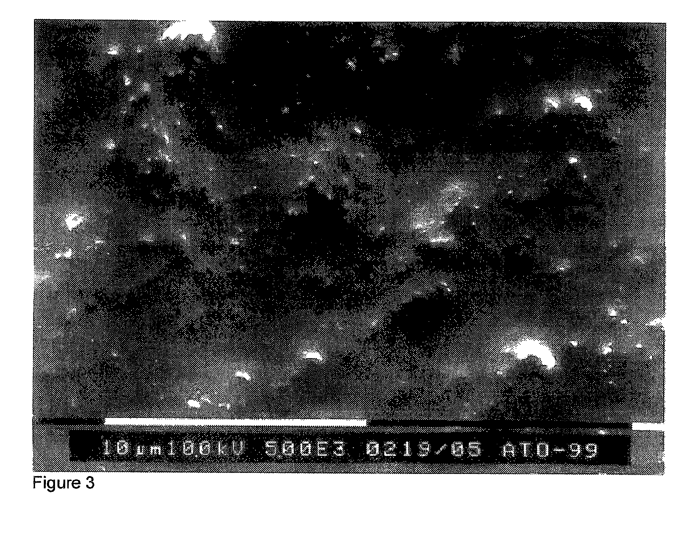

The process according to the invention for encapsulating liquid, hydrophobic active ingredients in a moderately to strongly hydrophilic matrix has significant advantages over conventional processes. In the first place, the size of the inclusions containing the active ingredients, can be precisely controlled, largely independently of the processing conditions in the extruder. Whereas in conventional extrusion encapsulation, inclusion sizes below 5 mm to 10 mm are very difficult to realize, according to the invention inclusions of sizes below 2 mm, preferably below 1 mm are obtained. Inclusions smaller than about 0.01 mm to 0.05 mm do not significantly contribute to the total amount of encapsulated oil. This is very difficult to achieve using conventional extrusion encapsulation techniques. Accurate knowledge about the inclusion size in the extrudate can be obtained by measuring the droplet size distribution of the emulsion, for instance by scattering methods. In the second place, the evaporation of the active ingredients during the encapsulation process is strongly reduced because the active ingredients are protected by an emulsifier film when they are introduced into the matrix. Introduction of the emulsion via a feed port mounted on the extruder barrel gives a high flexibility with respect to the processing of the matrix composition, while at the same time limiting the evaporation and degradation of active ingredients. In the third place, the stability of the active ingredients in the extruded product is very high because the small size of the inclusions and because the inclusions are fully covered by an emulsifier film. The emulsifier is used more efficiently when it is added to the matrix premix. In the fourth place, gentle mixing conditions in the extruder barrel will suffice to uniformly disperse the emulsion droplets through the matrix, in strong contrast with conventional extrusion encapsulation techniques, where high shearing forces are necessary, even to achieve a rather coarse dispersion quality. As is well-known, high shearing forces may unnecessarily degrade polymeric matrix constituents. In the fifth place, the range of matrix materials which can be used is increased because viscosity disparities between the high-viscous matrix and the low-viscous liquid active ingredients are minimized and the affinity of the hydrophobic active ingredient for the hydrophilic matrix is increased through the use of an emulsifier film. A sixth advantage is that the amount of surface oil upon fracture of the extrudate is low because of the very small inclusion size (compare Figures 2 and 3). Inclusion sizes are shown in Figures 2 and 3. The electron micrograph of Figure 2 shows inclusion with a diameter of up to 5 micron, which is representative for extrudate according to the state of the art. Further a broad size distribution is shown as well. In Figure 3 he extrudate according to the invention are significantly smaller than 1 micron. Further an almost monodisperse size distribution is shown.

### Caption to the figures

Figure 1 shows a process flow chart emphasizing the addition of active emulsion into the extruder barrel after matrix processing.

Figure 2 is an electron micrograph of fracture interface of cryogenically fractured extrudate according to the state of the art. Liquid orange flavor injected into the extruder barrel (load 10% w/w). Matrix composition: native potato starch, Capsul E (15 % w/w of dry starch) and glycerol (10% w/w of dry starch).

Figure 3 is an electron micrograph of fracture interface of cryogenically fractured extrudate according to the invention. Emulsified orange flavor injected in extruder barrel (emulsifier: Capsul E). Total load of orange flavor 10% w/w. Matrix composition: native potato starch, glycerol 10% w/w of dry starch).

However, it has also been surprisingly found that by the novel method according to the invention , the mechanical properties of the extruded product is modified, leading to a significantly reduced sensitivity of the encapsulated compounds to fracture of the extrudate. Upon fracture, a very large fraction of the droplets containing the active ingredients remains intact, limiting the amount of surface oil. This type of fracture behavior is not observed when other extrusion encapsulation techniques are used.

The properties of both the matrix and the dispersed phase may be optimized for a specific application. In general, the particles are prepared in such a way that they dissolve in a controlled manner in water or are water-insoluble, but slowly swelling in aqueous or humid environment, which makes the matrix permeable for the active ingredient. Due to this solubilization or swelling, the active ingredient is released in a controlled manner into the environment. In particular for flavors, the release is tuned such that either the main fraction of the flavor is released into a food product after processing or that the flavor is released from the composite material during consumption of the food product.

The extrudate may directly be used, or be subjected to a post-extrusion process to obtain the desired range in particle size. The post-extrusion process may be carried out in a state in which the extruded product is still moldable or in a state where it is brittle and shows glass-like behavior. Upon fracture of the extrudate, the amount of surface oil will be minimal, owing to the excellent encapsulation of the flavor droplets within the extrudate and the favorable breaking patterns, leaving the droplets on the interface intact.

The matrix material of which the particles are formed is a slightly to moderately hydrophilic polymer or oligomer, of biological or synthetic origin. The encapsulation matrix may consist of one polymeric or oligomeric compound, or of a mixture of two or more of those compounds. A convenient biopolymeric matrix material is native starch, originating from potato, maize or other vegetable sources. The starch may also be modified, either enzymatically or chemically, be fractionated according to molecular weight or be separated into its molecular constituents amylose and amylopectin. The modified starch may also be thermoplastic starch. Other polysaccharides, lignosulfonates and proteins may also be used. Apart from natural materials, thermoplastic synthetic polymers can be used. Some which are particularly useful are cellulose esters and ethers, polyvinylalcohol and its copolymers with vinyl acetate, (meth)acrylic acid and vinyl sulfonate, polyesters, sulfonated polyesters, polyvinylpyrrolidone, polyvinylmalonate, polyoxyalkylenes, polyhydroxyacids, polyacrylates and poly(styrol-co-acrylates). The matrix properties may be optimized by addition of plasticizers, antiplasticizers, fillers, crosslinking agents, compounds which influence the formation of crystallites or ordered regions in the polymeric or oligomeric materials and materials which promote the rapid dissolution of the matrix when immersed in aqueous environment.

Plasticizers are added to decrease the glass transition temperature (Tg) of the thermoplastic matrix material. By reducing the Tg, the processing temperature which is required is reduced and, if the plasticizers do not evaporate from the product during or after processing, the hardness and brittleness of the final product is reduced. Plasticizers may also bring additional processing benefits, like that they can act as lubricants and thereby reduce the friction between the extruder barrel and screw and the matrix. Typical plasticizers are low molecular compounds which are miscible with the matrix material. Depending on the matrix material used, the plasticizer can be selected from, but is not limited to water, alcohols, glycerin, glycols, polyalkylene glycols, dimethicones and polyether-, and/or alkyl-modified dimethicones and dialkylphtalates.

Also, materials may be mixed in, which have an effect on the absorption of water or other fluid compounds in the matrix, or have an effect on the barrier properties of the matrix with respect to oxygen, liquid water, water vapor and organic compounds, including the active ingredient or ingredients.

As emulsifying agent, a single emulsifier may be used, or a mixture of emulsifiers. Examples of food-grade emulsifiers which may advantageously be used are modified starches like Capsul and Hi-Cap (both supplied by National Starch), sucrose and sorbitol esters of fatty acids (Tween 20, Tween 60, Tween 80 (all supplied by ICI), Sisterna sucrose esters (supplied by Sisterna)), carbohydrates (e.g. gum arabic, pectin) and phospolipids (e.g. lecithin). For non-food applications, a much wider range of emulsifiers of natural or synthetic origin is available. Synthetic emulsifiers can be selected from monomolecular surfactants, polymer surfactants and colloid stabilisers. Typical monomolecular surfactants are alkyl sulfates, alkylethoxylates, alkylphenolethoxylates, alkylglucosides. Example of polymer surfactants are polyoxyethylene-polyoxypropylene-polyoxyethylene triblock-copolymers (Pluronic) and polyether modified dimethicones. Typical colloid stabilizers encompass partially hydrolyzed polyvinylalcohol, polyvinylpyrrolidon, cellulose and cellulose derivatives. If desired, a co-surfactant, selected from primary alcohols and short chain alkylsulfates or any compound having surface activity may be added to the emulsifier or emulsifiers to obtain optimal properties. Important criteria for the selection of a suitable emulsifier system are the emulsifying capacity of the emulsifier and the stability with respect to flocculation, creaming, coalescence and phase separation.

If desired, anti-oxidants, polymers, oils or other compounds may be added to the active ingredients. For instance, edible oils and other hydrophobic liquid compounds may be added to reduce the vapor pressure of the active ingredients. Edible oils which are often used for this purpose are sunflower oil, miglyol and arachid oil. Natural and synthetic oils and mixtures thereof with natural or synthetic polymers, waxes and resins, can be used to optimize retention of fragrance compounds. Examples of food-grade anti-oxidants which can be used to provide extra protection of oxidation-sensitive active ingredients are butylated hydroxyanisole (BHA) and vitamine E. An example of non-edible anti-oxydants is di(t-butyl)-hydroxy-toluene.

The extrudable premix consists of the composition of matrix materials, optionally solvent, which, in many cases, is water or another polar low-molecular weight compound, although nonpolar organic solvents may find application, and optionally additives. The matrix premix consists of 50 to 100% w/w polymeric constituents, 0 to 50% w/w plasticizing agents or solvents and 0 to 30% w/w additives. The additives can be fillers, plasticizers, antiplasticizers, crosslinking agents and agents which induce crystallization or a local ordering in the matrix, and any compounds which improve matrix properties with respect to hygroscopicity and barrier properties for oxygen and organic compounds. The total amount of active ingredient encapsulant in the product after processing is 1 to 50% w/w, preferably between 5 and 30 % w/w, with respect to the amount of dry, polymeric matrix constituents.

The premix is extruded in a conventional single or double screw extruder in a manner known per se.

The method according to the invention is particularly suited for the encapsulation of flavor components, compositions and extracts, fragrance components, odor masking agents, perfumes and precursors thereof, pharmaceutical compounds, agro-chemicals, cosmetic compounds and other chemical specialties, as long as they are moderately to strongly hydrophobic and can be emulsified.

The controlled release and delivery properties of encapsulated flavors may advantageously be used in dry soups and sauces, instant products, bakery products (cakes, bread specialties, bread mixes, pancake mixes, cake mixes), savory and meat products, pasta, hot and cold beverages (for instance flavored tea), pharmaceutical applications (powders, capsules and tablets), cereals, chewing gum and confectionary products. As the release of the encapsulated flavor is triggered by an increase of water activity, and optionally, an increase in temperature, successful application of flavors encapsulated following the method outlined here is restricted to products in which the water activity is moderately high or high during at least one stage of production, storage or consumption of the food product. Furthermore, for a complete release of the encapsulated flavor, the state of moderately high or high water activity should be maintained for a certain minimum period.

Fragrance compounds or perfumes may also successfully be encapsulated using the present method. The encapsulation system is useful in the protection of fragrances in dry detergent powders, in house-hold products and in cosmetic applications. For instance, in a powder detergent application, the matrix slowly swells and/or dissolves during the wash-cycle, gradually releasing the perfume over time. A beneficial controlled release may also be obtained in other house-hold applications like toilet blocks and in cosmetic and personal care products.

Agro-chemicals, biologically active compounds and other specialty chemicals and biochemicals, which are encapsulated following the present method may be successfully used in controlled release applications. For instance, pesticides, insecticides, herbicides, fungicides or insect repellents may be encapsulated for use in crop protection, wood protection, textile protection and household applications. Pharmaceutical compounds may be encapsulated for pharmaceutical and medicinal applications.

A most useful application is the encapsulation of two or more emulsions in a single matrix, each of the dispersed phases containing a hydrophobic chemical agent which would chemically react with one or more of the components present in one or more of the other dispersed phases. The migration of the reactive chemical compounds in the matrix is prohibited by the matrix and the emulsifier film during storage; upon use in the final product, both components are gradually released. In this way, in a convenient single component product, two or more reactive components together bringing desirable properties like biologial functioning, cleaning properties, taste and smell can be brought together. For instance, two reactive flavors may in this way be sucessfully encapsulated.

### Example 1

A matrix premix is prepared by mixing 2000 g native potato starch (Avebe, the Netherlands) with 85 g glycerol (Chemproha B.V.) and 600 g water. The water content of the potato starch was determined to be 17% by weight, leading to a water content of 31% by weight in the premix. Equivalently, the weight ratio of water to dry starch is 0.57, for which the maximum degree of gelatinization is about 40%. An orange flavor emulsion was prepared by mixing 180 g orange flavor BC2420 (Givaudan Roure) with 75 g Capsul E (National Starch) and 180 g water. Emulsification was carried out by blending at maximum speed with an Ultra-Turrax T25 high-speed homogenizator (IKA Labortechnik) for approximately 10 minutes, while cooling the emulsion mixture by placing its container in an ice bath. The droplet size of the emulsion was typically smaller than 1 µm, as determined on a Mastersizer Longbench particle sizer (Malvern).

The extrusion was carried out on a Berstorff ZE25 twin-screw, co-rotating extruder. The diameter of the extruder barrel is 25 mm and the length is 100 cm. The extruder screws were build up of small and large forward transport elements, backward transport elements, mixing elements and forward and backward kneading elements. The emulsion injection port was situated approximately 25 cm from the die of the extruder barrel. Immediately after the injection port, the extruder screw contained a mixing element to ensure proper mixing of the injected emulsion with the matrix. The temperature profile over the extruder barrel varied between 70°C and 120°C, the temperature increasing in steps of 10°C from 70°C near the feed opening to 120°C halfway the barrel, and deceasing to 60 to 70 °C at the flavor injection point and reaching 50 to 70°C near the die (diameter 7 mm). The throughput of the extruder was 3.5 kg/h, with a die pressure of 21 bar.

Immediately after extrusion, the extrudate was collected in an aluminium tray and stored at 30% RH for about two weeks. Afterwards, the extrudate was ground using a Braun 4214 blender.

### Example 2

Hot beverages were prepared by adding 200 ml of boiling water to standardized amounts of flavor and flavor encapsulates: sample A containing 0.05 g liquid flavor BC 2420 (Givaudan Roure, the Netherlands), sample B containing 0.2 g Flav-O-Lok CB 2774 (Givaudan Roure, the Netherlands) and sample C containing 0.6 g starch extrudate of example 1, with particle sizes between 500 and 600 mm. The intensity and quality of smell and taste were observed over time.

Sample A: Instantaneous very strong impact, decreasing in time to about one-tenth of the strength after 10 minutes, after which the smell and taste revealed the presence mainly of terpenes. After three days, no orange flavor could be detected sensorically.

Sample B: Increasing in strength during the first and second minutes, remaining strong for about ten minutes, after which a gradual decrease in strength was observed. The quality of the orange flavor changed from a fresh orange impact to somewhat terpene-like. After three days, a very weak citrus scent could still be detected.

Sample C: Weak impact giving the impression of fresh orange juice during the first minutes; steadily increasing over time and becoming stronger than sample B after 10 to 15 minutes. Still increasing in strength after half an hour. After three days, the sample still had a weak but reasonably balanced orange taste and smell.

### Example 3

Washing tests were performed using a liquid detergent perfume, a spraydried version of the same perfume and an perfume encapsulated in native potato starch by the method of Example 1. 80 g of neutral powder detergent base was mixed with the equivalent amount of 0.4 g of detergent perfume: sample A 0.4 g liquid perfume, sample B 1.6 g spraydried perfume and sample C 8 g of starch extrusion encapsulated perfume. For each perfume system, eight cotton towels (25 cm x 25 cm) were washed at 60°C during 40 minutes in a Shulthess Super 45 washing machine. Four towels were taken out of the washing machine before rinsing, centrifuged for 1 minute in a Bauknecht WS130 and put to dry on a drying rack. The other four were rinsed, centrifuged as decribed above, and put to dry on a drying rack.

The strength of the wet, unrinsed sample towels was sensorically assessed as follows (from strong (++++) to weak (+)):

| | Sample A | Sample B | Sample C |
|---|---|---|---|
| Unrinsed, wet | +++ | +++ | ++++ |
| Unrinsed, dry | ++ | ++ | +++ |
| Rinsed, wet | ++ | ++ | +++ |
| Rinsed, dry | (+) | (+) | +(+) |

### Example 4

The quality of the encapsulated dispersion was assessed by comparing the levels of surface oil of the extrudate of Example 1 with an extrudate in which an equivalent amount of orange flavor was encapsulated in liquid form.

The latter sample was prepared by extruding a premix consisting of 2000 g native potato starch (watercontent 17%) (Avebe, the Netherlands) with 165 g glycerol (Chemproha B.V., the Netherlands), 250 g Capsul E (National Starch, USA) and 600 g water. The temperature profile over the extruder barrel was the same as in Example 1. During extrusion, 200 g of Orange flavor BC2420 was injected into the proccessed matrix through the injection port close to the extruder die.

Immediately after extrusion, the extrudate was collected in an aluminium tray and stored at 30% RH for about two weeks. Afterwards, the extrudate was ground using a Braun 4214 blender.

Small quantities of the ground extrudates (emulsion injection and liquid flavor injection) were fractionated according to partcle sizes with a set of analytical sieves (10 cm diameter; particle sizes 200-500 mm). After extraction, several of the fractions of both extrudates were analyzed for surface oil by a pentane extraction. For this, 5 g ground and fractionated extrudate was mixed with 40 ml pentane (analytical grade) and filtered over a standard glass filter. The extract was collected in a bottle. the residue on the glass filter was rinsed twice with 20 ml pentane, which was also collected in the bottle. Afterwards, the extract was analyzed according to standard GC procedures for flavor content. The found levels of surface oil were normalized for the flavor load of the extrudate and expressed in g per 100 g of total extrudate at a flavor load of 10% of the dry matrix material.

| **Surface oil** | **particle size 200 - 250 mm** | **Particle size 400-450 mm** |
|---|---|---|
| (g/100 g extrudate (10% load) | | |
| Extrudate (emulsion injected) | 0.17 | 0.13 |
| Extrudate (liquid injected) | 0.7 | 0.4 |

Clearly, the use of an emulsion instead of a liquid flavor substantially reduces the fraction of surface oil, in our example by a factor of 3.

### Example 5

An extrusion premix was prepared with 2000 g of native potato starch (Avebe, the Netherlands) with 85 g glycerol (Chemproha B.V., the Netherlands) and 600 g of water. Three emulsions were prepared, one with Premix Beef 581411H without miglyol and palmatic acid (Givaudan Roure, the Netherlands), one with onion flavor 580943H without miglyol (Givaudan Roure, the Netherlands) and one with a 50/50% by weight mixture of the beef premix and onion flavor. For each emulsion, 150 g of water was mixed with 150 g flavor and 50 g Capsul E. The emulsification was carried out by blending at full speed during 10 minutes with a Ultra-Turrax T25 homogenisator (IKA Ruhrwerke).

The extrusion was carried out as in Example 1. During the first part of the run, the beef/onion flavor was injected in the extruded matrix, whereas during the second part of the run, a mixture of the beef emulsion and the onion emulsion was injected. For the second part of the run, the beef emulsion and the onion emulsion were mixed just prior to the injection step, to avoid migration of components of the beef flavor to the onion emulsion droplets and vice-versa. The effective loading of the extrudate with flavor was approximately 8% by weight.

The extrudate was rapidly cooled down to room temperature and stored for three weeks at 30% RH.

Both samples were evaluated by a panel of three flavorists. Equal amounts of ground extrudate were suspended in cold water and evaluated with respect to smell. A clear distinction could be made between the samples, with the sample containing the beef/onion emulsion having a sharper smell than the sample containing the separate beef and onion emulsions. The sample containing the beef and the onion emulsions was preferred by all three flavorists, as both the onion and the beef notes could be clearly discerned.

The controlled release properties of the sample containing the beef emulsion and the onion emulsion were evaluated in a comparison with Flavorburst coacervates (Givaudan Roure, USA) loaded with the same flavors at a level of 2.5% by weight.

1.5 g of a 50/50 % mixture by weight of the Flavorburst loaded with onion flavor and the Flavorburst loaded with beef flavor and 0.5 g of the ground beef emulsion/onion emulsion extrudate were each suspended in 500 ml of a standard stock (Givaudan Roure Flavors, Switzerland). Both the stocks containing the flavor encapsulates and 500 ml of the standard stock were brought to the boil. After 5, 10 and 20 minutes of continuous boiling, samples were taken for a sensory evaluation by a panel of four panelists. In the table below, the results of the sensory evaluation are summarized.

| **Time** (min) | **Starch extrudate** | **Flavorburst** |
|---|---|---|
| 5 | Strong beef and onion smell and taste. | Strong beef smell and taste. Weak impression of onion. |
| 10 | Very strong impression of beef. Reasonably strong onion smell and taste. | Strong beef smell and taste. No clear onion note discernible. |
| 20 | No clear onion impression. Moderately weak impression of beef. | No clear impression of onion. Weak impression of beef. |

From this evaluation, it is clear that encapsulation in starch has improved controlled release characteristics with respect to Flavorburst. Moreover, the onion and beef notes of the starch encapsulate were perceived as fresh and natural, demonstrating the stability of the two flavors encapsulated in a single matrix.

## Claims

1. A composite material comprising a thermoplastic hydrophilic matrix in which a liquid active ingredient in form of a water-in-oil emulsion is encapsulated in the form of very fine and uniformly distributed droplets.

2. A composite material according to claim 1 characterised in that the inclusions have a droplet size between 0.01 µm to 2 µm, preferably between 0.05 µm and 1 µm.

3. A composite material according to any of the preceding claims, characterised in that the load of active ingredients in the composite material amounts to 1 to 50 % w/w preferably to 5 to 15% w/w.

4. A composite material according to any of the preceding claims, characterised in that the active ingredient is a flavor compound, extract, precursor or a composition containing a flavor compound.

5. A composite material according to any of the claims 1 to 3, characterised in that the active ingredient is a fragrance, fragrance precursor or an odor masking agent.

6. A composite material according to any of the claims 1 to 3, characterised in that the active ingredient is a compound with a biological activity such as a pharmaceutically active substance, an insect repellent, a bactericide, a fungicide or an accaricide.

7. A composite material according to any of the preceding claims, comprising droplets of different composition.

8. Method for preparation of composite material according to any of the claims 1 to 7 characterised in that a liquid active ingredient in form of a water-in-oil emulsion is mixed with a matrix premix and the mixture obtained extruded.

9. Method according to claim 8, characterised in that the emulsified liquid, hydrophobic active ingredient is mixed with the matrix premix and the mixture is introduced into the extruder.

10. Method according to claim 8, characterised in that the emulsion of the liquid, hydrophobic active ingredient is introduced into the barrel of an extruder, containing the pre-processed matrix composition, and the emulsion droplets are mixed with the matrix composition.

11. Method according to any of the claims 8 to 10, characterised in that the amount of polymeric fraction in the matrix premix corresponds to 50% w/w to 100% w/w.

12. Method according to any of the claims 8 to 11, characterised in that the oil in water emulsion contains 5 to 80% w/w, preferably 30 to 60% w/w active ingredients, 10 to 90% w/w, preferably 15 to 40% w/w water, 0,5 to 10% w/w emulsifier and 0 to 10% w/w additives

13. Method according to any of the claims 8 to 12, characterised in that the matrix comprises one or more hydrophilic thermoplastic polymers, selected from native starch, modified starch, thermoplastic starch, polyvinylalcohol and its copolymers and polyesters and one of more additives like crosslinking agents, plastifiers, antiplastifiers, fillers, compounds which influence the formation of crystallites and ordered regions in the matrix.

14. Method according to any of the claims 8 to 13, characterised in that the emulsifier used to prepare the emulsion is a modified starch, a sucrose or sorbitol ester of a fatty acid, a carbohydrate, a phospholipid, a monomolecular surfactant, a polymeric surfactant or a colloid stabilizer, a co-surfactant, selected from primary alcohols, and short chain alkylsulfates or any compound having surface activity.

15. Use of the composite material according to claims 1 to 7 as protective or controlled release system for flavor compositions.

16. Use of the composite material according to claims 1 to 7 as protective or controlled release system for fragrance compositions in dry detergent, household products and cosmetic applications.
